# EUROPÄISCHE PATENTSCHRIFT

(11) **EP 1 154 980 B1**
(45) Veröffentlichungstag und Bekanntmachung des Hinweises auf die Patenterteilung: **26.05.2004**
(21) Anmeldenummer: 00902603.0
(22) Anmeldetag: 18.01.2000
(51) Int. Cl.: C07C 68/04, C07C 69/96

(54) **VERFAHREN ZUR HERSTELLUNG VON SYMMETRISCHEN UND UNSYMMETRISCHEN CARBONATEN**
METHOD FOR PRODUCING SYMMETRICAL AND ASYMMETRICAL CARBONATES
PROCEDE DE PREPARATION DE CARBONATES SYMETRIQUES ET ASYMETRIQUES

(30) Priorität: 09.02.1999 DE 19905222
(43) Veröffentlichungstag der Anmeldung: 21.11.2001
(73) Patentinhaber: GÖDECKE GMBH, 76139 Karlsruhe (DE)
(72) Erfinder: BARTH, Hubert, D-79312 Emmendingen (DE); STEINER, Klaus, D-79312 Emmendingen (DE); SCHNEIDER, Simon, D-79249 Merzhausen (DE); BAYER, Ulrich, D-89077 Ulm (DE); WESTERMAYER, Manfred, D-79194 Gundelfingen (DE); WOLFSPERGER, Ulrike, D-79194 Gundelfingen (DE)
(74) Vertreter: Tesch, Rudolf, Dr.
(86) Internationale Anmeldenummer: PCT/EP2000/000340
(87) Internationale Veröffentlichungsnummer: WO 2000/047544

(56) Entgegenhaltungen:
- FANG, SHUNNONG ET AL: "Direct synthesis of dimethyl carbonate from carbon dioxide and methanol catalyzed by base" APPL. CATAL., A (1996), 142(1), L1-L3 , XP000882681
- BUTCHER ET AL: "Carbamate esters: a simple, mild method of formation" SYNLETT, XP000882931 in der Anmeldung erwähnt
- DATABASE WPI Section Ch, Week 199515 Derwent Publications Ltd., London, GB; Class A41, AN 1995-110592 XP002134803 & JP 07 033715 A (SHOWA DENKO KK), 3. Februar 1995 (1995-02-03)

## Beschreibung

Die vorliegende Erfindung betrifft die Herstellung von symmetrischen und unsymmetrischen Carbonaten der allgemeinen Formel I 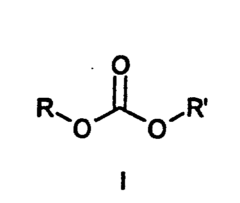 worin R und R' gleich oder verschieden sind und eine geradkettige oder verzweigte Alkylgruppe mit 1 bis 10 C-Atomen, eine unsubstituierte oder mit bis zu drei C₁-C₄-Alkylgruppen, C₁-C₄-Alkoxygruppen, Halogenatomen, mit einer Cyanogruppe, einer Nitrogruppe, einer Trifluormethylgruppe oder einer Alkoxycarbonylgruppe mit bis zu 4 C-Atomen substituierte Benzylgruppe, eine Aralkylgruppe oder eine Alkenylgruppe bedeuten. Der Begriff Aralkylgruppe umfaßt einen niederen Alkylrest mit 2 bis 10 C-Atomen, worin bis zu zwei H Atome durch Phenylgruppen ersetzt sind, die gegebenenfalls wiederum mit einer C₁-C₄-Alkylgruppe, einer C₁-C₄-Alkoxygruppe, einer Cyanogruppe, einer Nitrogruppe, einer Trifluoromethylgruppe, einer Alkoxycarbonylgruppe mit bis zu 4 C-Atomen oder mit bis zu drei Halogenatomen substituiert sein können. Der Begriff Alkenyl bezeichnet einen ungesättigten Kohlenwasserstoffrest mit bis zu 5 C-Atomen.

Organische Carbonate spielen eine wichtige Rolle als Lösungsmittel, als Zwischenprodukte für zahlreiche Synthesen und als Produkte für spezielle Anwendungsgebiete, z.B. in der Agrikulturchemie oder der Medizinischen Chemie (Ullmann's Encyclopedia of Industrial Chemistry, 5^{th} Ed., Vol. A5, S. 197, 1986; KIRK-OTHMER, Encyclopedia of Chemical Technology, 3^{th} Ed., Vol. 4, S. 766, 1978; Abbas-Alli G. Shaikh, Chem. Rev. 1996, 96, 951-976). Die Herstellung offenkettiger organischer Carbonate kann z.B. erfolgen (i) aus Phosgen und Hydroxyverbindungen, (ii) aus Halogenameisensäuren durch Umsetzung mit Hydroxyverbindungen, (iii) durch Alkylierung von Alkalimetallcarbonaten (iv) durch Umesterung von Kohlensäurediestern oder (v) aus Kohlendioxid und Alkoholen unter Druck in Gegenwart von Katalysatoren oder aber nach anderen speziellen Verfahren (H. Hagemann, HOUBEN WEYL, E4, S. 65, 1983; Abbas Alli G. Shaikh, Chem. Rev. 1996, 96, 951; S. Fang, K. Fujimoto, Applied Catalysis A: General 142 (1996) L1-L3. Verfahren zur Herstellung von organischen Carbonaten, die die Verwendung des hochgiftigen Phosgens vermeiden, das vorhandene Kohlendioxid nutzen können und von einfachen Rohstoffen ausgehen, sind aus industrieller sowie organisch-präparativer Sicht von besonderem Interesse. Angeregt durch eine Arbeit von Ken J. Butcher zur Herstellung von Carbamaten aus Aminen und Kohlendioxid (Ken J. Butcher, Synlett 1994, 825) wurde von uns untersucht, ob sich Alkohole der allgemeinen Formel II unter Verwendung von Kohlendioxid, Cesiumcarbonat und Alkyl- bzw. Arylhaliden der allgemeinen Formel III, 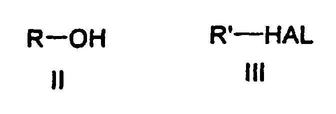 wobei R und R' die oben genannte Bedeutung besitzen und HAL für Chlor, Brom oder Iod steht, in organische Carbonate der allgemeinen Formel I überführen lassen (Schema 1): 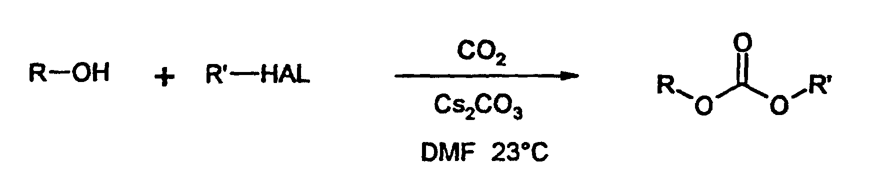 Aufgrund der geringeren Nucleophilie der OH-Gruppe in Alkoholen im Vergleich zur NH₂-Gruppe in Aminen und aufgrund der in der Literatur beschriebenen speziellen Methoden zur Herstellung von Carbonaten aus Kohlendioxid (Abbas-Alli G. Shaikh, Chem. Rev. 1996, 96, 951) war ein synthetischer Zugang zu Carbonaten unter Verwendung des Systems Kohlendioxid/Cesiumcarbonat bei niedrigen Temperaturen nicht zu erwarten.

überraschenderweise wurde jedoch gefunden, daß sich organische Carbonate der allgemeinen Formel I unter sehr milden und präparativ einfachen Bedingungen in Gegenwart von Cesiumcarbonat aus Alkoholen der allgemeinen Formel II und Alkyl- bzw. Arylhalogeniden der allgemeinen Formel III herstellen lassen.Für diese Reaktion ist überraschenderweise kein weiterer Katalysator erforderlich. Die präparative Vorgehensweise ist wie folgt:

Der Alkohol und ein 2 bis dreifacher molarer Überschuss an Cesiumcarbonat werden in einem geeigneten dipolar aprotischen Lösungsmittel wie z.B. Dimethylformamid, Acetonitril, Dimethylacetamid oder N-Methylpyrrolidon bei Raumtemperatur vorgelegt. Unter gutem Rühren wird nun bei Raumtemperatur unter Feuchtigkeitsausschluß 4 bis 6 Stunden Kohlendioxidgas in die Reaktionsmischung eingeleitet (ca. 5 Blasen/Sekunde). Das Kohlendioxid wird hierbei durch Verdampfenlassen von Trockeneis erzeugt, das sich in einem Erlenmeyerkolben befindet, der mit dem Reaktionsgefäß über ein Gaseinleitungsrohr verbunden ist. Nun fügt man in einer Portion 1 Equivalent (bezogen auf den Alkohol) des betreffenden Alkyl- bzw. Aryl-halogenids der allgemeinen Formel III, gelöst in wenig Lösemittel, zur Reaktionsmischung, leitet 1 Stunde weiter Kohlendioxid ein , fügt nochmals 5-100%, vorzugsweise 10%, der ursprünglichen Alkyl- bzw. Arylhalogenidmenge hinzu und verschließt dann das Reaktionsgefäß. Bei geschlossenem Reaktionsgefäß rührt man nun 24 Stunden bis 3 Tage bei Raumtemperatur weiter. Danach gießt man die Reaktionsmischung auf Wasser, extrahiert das Produkt mit Essigester und reinigt das so erhaltene Rohprodukt mit den in der präparativen organischen Chemie üblichen Methoden, z.B. durch Chromatographie oder Kristallisation. Bevorzugtes Lösungsmittel für die beschriebene Reaktion ist Dimethylformamid.
Die Reaktionsbedingungen sind sehr milde, es werden viele funktionelle Gruppen, wie z.B. die Doppelbindung, die Nitrogruppe, die Alkoxycarbonylgruppe, die Cyanogruppe, Halogengruppen und Alkoxygruppen an Aromaten toleriert. Die Ausgangsmaterialien, - Alkohole und Alkyl- und Arylhalogenide - sind einfach herstellbar und stehen in großer Zahl käuflich zur Verfügung. Sehr einfach gestalten sich die Bedingungen zum Aufarbeiten der Reaktion.
Unter der Annahme, daß Cesiumcarbonat aus dem extrahierten, wässrigen Rückstand wiederhergestellt werden kann, ist die Methode geeignet, gasförmiges Kohlendioxid an einfache käufliche Ausgangsmaterialien wie Alkohole und Alkyl- bzw. Arylhalogenide zu binden und dadurch wertvolle, energiereiche Zwischenprodukte zu erzeugen.
In diesem Sinne ist das genannte Verfahren ein wertvoller Beitrag zu einer umweltfreundlichen Chemie.

Wegen der Einfachheit des Verfahrens ist die Vorgehensweise auch hervorragend als Basis für eine 'High Throughput Synthesis geeignet. Hierzu müßten in einer Kohlendioxid-Begasungs-Apparatur, die DMF-Lösungen entsprechender Alkohole enthalten, gleichzeitig mit CO₂ für einige Stunden begast werden. Danach sind die entsprechenden Alkyl- bzw. Arylhalogenide zuzudosieren, die Gefäße zu verschließen und für 24 Stunden bis 3 Tage bei Raumtemperatur zu rühren. Danach sind die gebildeten Carbonate in einfacher Weise zu isolieren.

Die Erfindung wird durch die nachfolgenden Ausführungsbeispiele veranschaulicht und erläutert.

### Beispiel 1

### Dibenzylcarbonat aus Benzylalkohol und Benzylbromid

In eine Suspension von 0.45 g Benzylalkohol, und 3.0 g Cesiumcarbonat in 30 mL trockenem Dimethylformamid, die sich in einem 50 mL Dreihalskolben befindet, wird 4 Std. unter gutem Rühren bei Raumtemperatur Kohlendioxidgas eingeleitet. Man fügt 0.7 g Benzylbromid, gelöst in wenig DMF, hinzu, leitet 1 Std. weiter Kohlendioxid ein, versetzt nochmals mit 0.1 g Benzylchlorid und verschließt dann luftdicht das Reaktionsgefäß. Die Reaktionsmischung wird nun 2 Tage bei Raumtemperatur weitergerührt. Danach gießt man die Reaktionsmischung auf 50 mL Wasser (Vorsicht: exotherme Reaktion) und extrahiert das Produkt 3 mal mit jeweils 50 mL Essigester. Die organische Phase wird über Natriumsulfat getrocknet, filtriert und am Rotavapor eingeengt. Das im öligen Rückstand zusammen mit dem Produkt befindliche Dimethylformamid wird am Rotavapor durch azeotrope Destillation mittels Toluol bei 40 mbar/50°C entfernt. Der Rückstand wird an 130 g Silica Gel (0.040 - 0.063 mm) mit Toluol als Elutionsmittel chromatographiert.
Man erhält
0.95 g Produkt, Fp. 30 - 31°C.

Die folgenden Beispiele wurden in Analogie zu Beispiel 1 durchgeführt (Reaktionszeit in Stunden/Ausbeute):

### Beispiel 2

### Benzyl-2-phenylethylcarbonat, Öl aus 2-Phenylethanol und Benzylbromid 48/93%

### Beispiel 3

### Benzyl-ethylcarbonat, Öl, aus Benzylalkohol und Ethylbromid 18/73%

### Beispiel 4

### Benzyl-tert.Butylcarbonat, Öl, aus tert.Butanol und Benzylbromid 120/15%

### Beispiel 5

### Di-Benzo[b]furan-2-yl-methylcarbonat, Öl aus 2-Hyrdroxymethylbenzo[b]furan und 2-Chlormethylbenzo[b]furan 120/23%

### Beispiel 6

### Benzyl-3-phenylpropylcarbonat, Öl aus 3-Phenylpropanol und Benzylbromid 120/99%

### Beispiel 7

### Benzyl-4-Chlorbenzylcarbonat, Öl aus Benzylalkohol und 4-Chlorbenzylchlorid 64/50%

### Beispiel 8

### Benzyl-4-Methoxybenzylcarbonat, Öl aus Benzylalkohol und 4-Methoxybenzylchlorid 88/64.4%

### Beispiel 9

### Benzyl-4-Methylbenzylcarbonat, Öl aus Benzylalkohol und 4-Methylbenzylchlorid 64/52.3%

### Beispiel 10

### Benzyl-2,4-Dichlorbenzylcarbonat, Öl aus Benzylalkohol und 2,4-Dichlorbenzylchlorid 64/49%

### Beispiel 11

### 4-Chlorbenzyl-2-phenylethylcarbonat, Öl aus 2-Phenylethanol und 4-Chlorbenzylchlorid 64/32.7%

### Beispiel 12

### Di-4-Methoxybenzylcarbonat, Fp 73°C aus 4-Methoxybenzylalkohol und 4-Methoxybenzylchlorid 88/72%

### Beispiel 13

### Di-2,4-Dichlorbenzylcarbonat, Öl aus 2,4-Dichlorbenzylalkohol und 2,4-Dichlorbenzylchlorid 64/70.5%

### Beispiel 14

### Di-4-Methylbenzylcarbonate, Fp 55°C aus 4-Methylbenzylalkohol und 4-Methylbenzylbromid 88/40%

### Beispiel 15

### Di-4-Chlorbenzylcarbonat, Fp 94°C aus 4-Chlorbenzylalkohol und 4-Chlorbenzylbromid 64/78.3%

### Beispiel 16

### Di-4-Chlorbenzylcarbonat, Fp 97°C aus 4-Chlorbenzylalkohol und 4-Chlorbenzylchlorid 64/54.8%

### Beispiel 17

### (±)-Benzyl-2-Methyl-2-phenylethylcarbonat, Öl aus (±)-2-Methyl-2-phenylethylalkohol und Benzylbromid 64/63.1%

### Beispiel 18

### Benzhydryl-Benzylcarbonat, Fp 72°C aus Benzhydrol und Benzylbromid 64/71.2%

### Beispiel 19

### (±)-Benzyl-1-Phenylethylcarbonat, Öl aus (±)-1-Phenylethanol und Benzylbromid 64/57.1%

### Beispiel 20

### Benzyl-3-Phenylpropylcarbonat, Öl aus 3-Phenylpropanol und Benzylbromid 120/99%

### Beispiel 21

### (±)-Benzyl-1-Methyl-2-phenylethylcarbonat, Öl aus (±)-1-Phenyl-2-propanol und Benzylbromid 120/99%

### Beispiel 22

### Benzyl-4-Methoxycarbonylbenzylcarbonat, Fp 53° aus 4-Methoxycarbonylbenzylalkohol und Benzylbromid 120/65%

### Beispiel 23

### Di-4-Nitrobenzylcarbonat, Fp 167° - 168°C aus 4-Nitrobenzylalkohol und 4-Nitrobenzylbromid 64/77%

### Beispiel 24

### Benzyl-benzo[b]furan-2-ylmethylcarbonat, Fp 59° - 60°C aus 2-Hydroxymethylbenzo[b]furan und Benzylbromid 64/100%

### Beispiel 25

### Benzyl-4-Cyanobenzylcarbonat, Fp 54° aus Benzylalkohol und 4-Cyanbenzylbromid 64/100%

### Beispiel 26

### Benzyl-3-Trifluormethylbenzylcarbonat, Öl aus Benzylalkohol und 3-Trifluormethylbenzylbromid 48/100%

### Beispiel 27

### Benzyl-1-Phenylethylcarbonat, Öl aus Benzylalkohol und 1-Phenylethylbromid 64/66%

### Beispiel 28

### Di-2-Phenylethylcarbonat, Fp 56°C aus 2-Phenylethanol und 2-Phenylethylbromid 64/69.4%

### Beispiel 29

### Di-3-Phenylpropylcarbonat, Öl aus 3-Phenylpropanol und 3-Phenylpropylbromid 64/98%

### Beispiel 30

### Benzyl-tert.Butylcarbonat, Öl aus Benzylalkohol und tert.Butylbromid 64/7%

### Beispiel 31

### Benzyl-4-Nitrobenzylcarbonat, Fp 68°C aus Benzylalkohol und 4-Nitrobenzylbromid 64/93%

### Beispiel 32

### Allyl-Benzylcarbonat, Öl, aus Benzylalkohol und Allylbromid 64/80%

### Beispiel 33

### Allyl-Benzylcarbonat, Öl aus Allylalkohol und Benzylbromid 64/70%

### Beispiel 34

### Benzyl-Cinnamylcarbonat, Öl aus Zimtalkohol und Benzylbromid 64/74.5%

### Beispiel 35

### (±)-Benzyl-1-Methylpropylcarbonat, Öl aus (±)-1-Methylpropanol und Benzylbromid 64/58%

### Beispiel 36

### Benzyl-Butylcarbonat, Öl aus n-Butanol und Benzylbromid 48/58.5%

### Beispiel 37

### Benzyl-4-Nitrobenzylcarbonat, Fp 68°C aus 4-Nitrobenzylalkohol und Benzylbromid 64/80.6%

## Patentansprüche

1. Verfahren zur Herstellung von symmetrischen und unsymmetrischen Carbonaten der allgemeinen Formel I 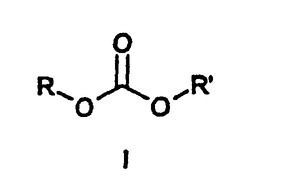 in welcher
R und R' gleich oder verschieden sind und eine geradkettige oder verzweigte Alkylgruppe mit 1 bis 10 C-Atomen, eine unsubstituierte oder mit bis zu drei C₁-C₄-Alkylgruppen, C₁-C₄-Alkoxygruppen, Halogenatomen, mit einer Cyanogruppe, einer Nitrogruppe, einer Trifluormethylgruppe oder einer Alkoxycarbonylgruppe mit bis zu 4 C-Atomen substituierte Benzylgruppe, eine Aralkylgruppe oder eine Alkenylgruppe bedeuten,
**dadurch gekennzeichnet, daß** man Alkohole der allgemeinen Formel II und Alkyl- bzw. Arylhalide der allgemeinen Formel III, 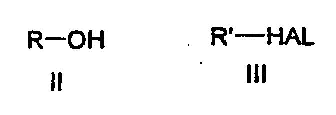 in welchen R und R' die oben genannte Bedeutung besitzen und HAL für Chlor, Brom oder Iod steht, mittels Kohlendioxid und Cesiumcarbonat in einem dipolar aprotischen Lösungsmittel in organische Carbonate der allgemeinen Formel I überführt.

2. Verfahren gemäß Anspruch 1, **dadurch gekennzeichnet, daß** das Lösungsmittel Dimethylformamid, Acetonitril, Dimethylacetamid oder N-Methylpyrrolidon ist.

3. Verfahren gemäß Anspruch 1, **dadurch gekennzeichnet, daß** die Reaktion bei Raumtemperatur durchgeführt wird.

4. Verfahren gemäß Anspruch 1, **dadurch gekennzeichnet, daß** das Kohlendioxid gasförmig in den Reaktionsansatz eingeleitet wird.

5. Verfahren gemäß Anspruch 1, **dadurch gekennzeichnet, daß** der Alkohol (II) mit einem 2- bis 3-fachen Uberschuss an Cesiumcarbonat in einem dipolar aprotischen Lösungsmittel vorgelegt wird, für mehrere Stunden Kohlendioxidgas eingeleitet wird und anschließend das Halid (III) equimolar zugesetzt und die Einleitung von Kohlendioxidgas noch für einige Zeit fortgesetzt wird.

## Claims

1. Method for the production of symmetric and asymmetric carbonates with the general formula I 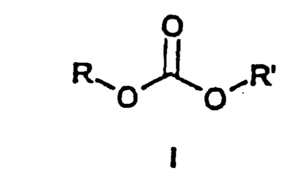 in which
R and R' are the same or different and stand for a straight-chain or branched alkyl group with 1 to 10 C atoms, for an unsubstituted benzyl group or a benzyl group substituted with up to three C1-C4-alkyl groups, C1-C4-alkoxy groups, halogen atoms, one cyano group, one nitro group or one trifluoromethyl group or with one alkoxycarbonyl group with up to 4 C atoms, or for an aralkyl group or an alkenyl group, **characterised in that** alcohols with the general formula II and alkyl or aryl halides with the general formula III, 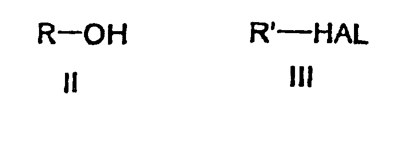 in which R and R' have the meaning attached to them above and in which HAL stands for chlorine, bromine or iodine, are reacted with carbon dioxide and caesium carbonate in a dipolar aprotic solvent to produce organic carbonates of the general formula I.

2. Method in accordance with Claim 1, **characterised in that** the solvent used is dimethyl formamide, acetonitrile, dimethyl acetamide or N-methyl pryrrolidone.

3. Method in accordance with Claim 1, **characterised in that** the reaction takes place at room temperature.

4. Method in accordance with Claim 1, **characterised in that** the carbon dioxide is introduced into the reaction starting mixture in gaseous form.

5. Method in accordance with Claim 1, **characterised in that** the alcohol (II) is used with a two- to three-fold excess of caesium carbonate as the starting mixture in a dipolar, aprotic solvent, that carbon dioxide gas is passed into the mixture for several hours and that the halide (III) is subsequently added in equimolar quantity, after which carbon dioxide continues to be passed into the mixture for some time.

## Revendications

1. Procédé de préparation de carbonates symétriques et asymétriques de formule générale : 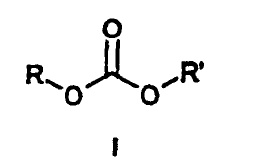 dans laquelle
R et R' sont identiques ou différents et représentent un groupe alkyle ramifié ou à chaîne droite ayant 1 à 10 atomes de C, un groupe benzyle non substitué ou substitué par un jusqu'à trois groupes alkyle en C₁ à C₄, groupes alcoxy en C₁ à C₄, atomes d'halogène, par un groupe cyano, un groupe nitro, par un groupe trifluorométhyle ou un groupe alcoxycarbonyle ayant jusqu'à 4 atomes de C, un groupe aralkyle ou un groupe alcényle,
**caractérisé en ce que** l'on transforme des alcools de formule générale II et des halogénures d'alkyle ou d'aryle de formule générale III, 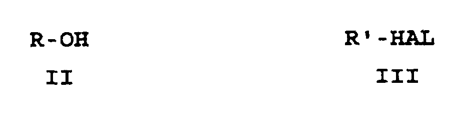 dans lesquelles R et R' possèdent la signification précitée et HAL représente un atome de chlore, de brome ou d'iode, en des carbonates organiques de formule générale I au moyen du dioxyde de carbone et du carbonate de césium dans un solvant aprotique dipolaire.

2. Procédé selon la revendication 1, **caractérisé en ce que** le solvant est le diméthylformamide, l'acétonitrile, le diméthylacétamide ou la N-méthylpyrrolidone.

3. Procédé selon la revendication 1, **caractérisé en ce que** la réaction est réalisée à la température ambiante.

4. Procédé selon la revendication 1, **caractérisé en ce que** le dioxyde de carbone est introduit dans le mélange réactionnel sous forme gazeuse.

5. Procédé selon la revendication 1, **caractérisé en ce que** l'alcool (II) est placé au préalable dans un solvant aprotique dipolaire avec un double ou triple excédent de carbonate de césium, du dioxyde de carbone gazeux y est introduit pendant plusieurs heures, puis l'halogénure (III) est ajouté de façon équimolaire et l'introduction du dioxyde de carbone gazeux est poursuivie encore pendant un certain temps.
